# EUROPEAN PATENT SPECIFICATION

(11) **EP 2 411 405 B1**
(45) Date of publication and mention of the grant of the patent: **09.09.2015**
(21) Application number: 10756825.5
(22) Date of filing: 25.03.2010
(51) Int. Cl.: C07K 1/28

(54) **ISOELECTRIC FOCUSING TRAY AND ELECTRODE ASSEMBLY FOR ALTERNATE GEL STRIP ORIENTATIONS**
TABLETT MIT ISOELEKTRISCHER FOKUSSIERUNG UND ELEKTRODENANORDNUNG FÜR ALTERNIERENDE GELSTREIFEN-AUSRICHTUNGEN
ENSEMBLE PLATEAU ET ÉLECTRODES D'ÉLECTROFOCALISATION POUR DES ORIENTATIONS ALTERNÉES DE LA BANDE DE GEL

(30) Priority: 25.03.2009 US 163289 P; 24.03.2010 US 730600
(43) Date of publication of application: 01.02.2012
(73) Proprietor: Bio-Rad Laboratories, Inc., Hercules, CA 94547 (US)
(72) Inventor: PEREZ, Evelio, Richmond CA 94894 (US)
(74) Representative: Von Kreisler Selting Werner - Partnerschaft von Patentanwälten und Rechtsanwälten mbB
(86) International application number: PCT/US2010/028595
(87) International publication number: WO 2010/111452

(56) References cited:
- EP-A1- 1 742 046
- WO-A1-99/33550
- US-A- 4 552 640
- US-A1- 2003 141 190
- US-A1- 2004 079 638
- US-A1- 2005 072 678
- US-B2- 6 932 895
- XU A ET AL: "Prototype for integrated two-dimensional gel electrophoresis for protein separation", JOURNAL OF CHROMATOGRAPHY, ELSEVIER SCIENCE PUBLISHERS B.V, NL, vol. 1087, no. 1-2, 16 September 2005 (2005-09-16), pages 177-182, XP027723800, ISSN: 0021-9673 [retrieved on 2005-09-16]

## Description

### BACKGROUND OF THE INVENTION

### 1. Field of the Invention

This invention resides in the field of laboratory equipment for electrophoretic separations, and the particular equipment addressed herein is that designed for isoelectric focusing.

### 2. Background of the Invention

As is well known in the art, isoelectric focusing is the separation of proteins into a linear array according to their isoelectric points and is performed on a sample for purposes of detection, analysis, and in some cases quantification of the proteins in the sample. Isoelectric focusing is typically performed in gel strips that contain immobilized pH gradients ("IPG strips"), and can serve either as the entire separation process of the proteins, or as the first dimension of a two-dimensional separation. In two-dimensional separations, the second dimension is performed by placing the IPG strip, after the first dimension separation has been performed, along one edge of a two-dimensional ("slab"-shaped) separation medium and imposing an electric field on the two-dimensional medium in a direction transverse to the IPG strip. This causes the proteins in each focused zone in the IPG strip to migrate. The proteins in the original sample thus have the benefit of being separated according to two parameters. Descriptions of isoelectric focusing and some of the equipment in which isoelectric focusing is performed can be found in Panattoni, "Assembly for Casting and Use of an Isoelectric Focusing Strip," United States Patent No. 6,655,649, issue date December 2, 2003; Williams et al., "Apparatus for Electrophoresis," United States Patent No. 6,558,522, issue date May 6, 2003 and WO99/33550, published July 8, 1999; Faupel et al., "Isoelectric Focusing Apparatus," United States Patent No. 5,082,548, issue date January 21, 1992; Witkorowicz et al., "Electrophoresis Apparatus and Method," United States Patent No. 6,214,191, issue date April 10, 2001; Zimmermann, "Lochable electrode for gel electrophoresis device", EP-A-1742046, published January 10, 2007; and Xu et al., "Prototype for integrated two-dimensional gel electrophoresis for protein separation", J. Chromatography 1087(1-2):177-182 (September 16, 2005).

IPG strips are commercially available from suppliers to biochemical laboratory suppliers and are commonly supplied in dehydrated form to be rehydrated by the user prior to use in the separation procedure. Both rehydration and focusing are typically performed in trays that accommodate as many as 24 IPG strips for simultaneous separations of as many samples. In some procedures, rehydration is performed in a rehydration/equilibration tray and once rehydrated, the strips are transferred to a separate focusing tray. In other procedures, rehydration and focusing are performed in the same tray. When the same tray, or any tray with electrodes contacting the strips, is used, rehydration can be performed in the presence of an electric current ("active rehydration"). This is particularly useful when the sample is combined with the rehydration solution and contains high molecular weight proteins, since the current enhances the entry of the proteins into the IPG strip. In certain separations, the sample is loaded through a separate receptacle at one end of the IPG strip, *i.e.,* by "cup loading," while in others the sample is applied to the entire length of the strip.

The typical IPG strip obtained from a supplier is a thin, flat strip of gel material that has a backing on one side for dimensional stability and ease of handling and a protective sheet on the other side. The protective sheet is removed by the user prior to rehydration and discarded, while the backing remains in place throughout both the rehydration and focusing. The strip thus contains a "gel side" and a backing side, and with the gel in a horizontal position the gel side can be facing either up or down during the rehydration step, and either up or down during the focusing step, depending on the needs of the sample to be separated, the preference of the user, or both. During focusing and active rehydration, however, the electrodes must be in contact with the gel side. The trays presently available are designed for use with gels in either one orientation or the other, *i.e.,* trays intended for contact of the electrodes with gels whose gel sides are facing up are distinct in their design and construction from trays intended for contact of the electrodes with gel sides facing down.

### SUMMARY OF THE INVENTION

The present invention resides in apparatus in which active rehydration, isoelectric focusing, or both can be performed either with the gel side of the IPG strip facing up and the electrode thus above the strip, or with the gel side facing down and the electrode thus beneath the strip, and with gels of a range of thicknesses in either orientation. This variability is achieved by constructing the tray and electrodes as separate components, *i.e.,* placing the electrodes in a removable frame, or preferably two such frames, one for each end of the tray. The electrodes in each frame terminate at their inner ends in exposed extensions, such as tabs or bars, that are either of metal or of a support material such as plastic with metal wires or strips on both their upper and lower surfaces. The tray contains a series of parallel channels or troughs, each sized to receive one IPG strip. The electrodes are mounted to the frame such that when the frame is in place in the tray, the exposed inner ends of the electrodes are inside the troughs, either in direct contact with, or close to, the floors of the troughs, with electrode material being exposed on both sides of each electrode end for contact with a gel either above or below the electrode. Further, said electrodes are mounted to said frame in a manner allowing for control of the height of each inner electrode end independently of all other inner electrode ends. Each exposed inner end of an electrode is sufficiently narrow to fit within one trough of the tray, and in preferred constructions, each frame contains slots between the electrode tabs to engage partitions that separate the troughs. The height of the exposed inner end of each electrode relative to the floor of each trough is manually adjusted in certain embodiments of the invention by user control of how far the frame is pushed into the tray. In other embodiments, the height of the exposed inner end of each electrode is automatically set by a spring-loaded or otherwise resilient mounting of the electrode, urging the exposed inner end toward the trough floor. With resiliently mounted electrodes, therefore, the height of each exposed inner end within its corresponding trough depends on whether a gel has been placed in the trough before or after the frame, and if before, on the thickness of the gel. Thus, when rehydration, isoelectric focusing, or both are to be performed with gel sides down, the frame is placed in the tray before the gels, and is inserted far enough that the inner electrode ends contact the floor of each empty trough. When the procedure is to be performed with gel sides up, the gels are placed in the troughs before the frames, and the frames are placed above the gels.

Among the advantages of this invention are the user's ability to use a single apparatus for active rehydration and isoelectric focusing in either orientation of the IPG strip, and to choose between loading the sample either by cup loading or by applying the sample to the entire IPG strip. Further advantages are the ability of the apparatus to accommodate IPG strips of both orientations at the same time by placing one or more strips in troughs of the tray before the frame is lowered into position and one or more other strips in other troughs of the same tray after the frame is lowered into position, and the ability of the apparatus to accommodate IPG strips of different thicknesses in a single tray.

These and other features, embodiments, and advantages of the invention will be apparent from the description that follows.

### BRIEF DESCRIPTION OF THE DRAWINGS

FIG. **1a** is a perspective view of one example of a tray and frame assembly in accordance with the present invention.
FIG. **1b** is a top view of the assembly of FIG. **1a****.**
**FIG. 1c** is a cross section of the assembly of FIGS. **1a** and **1b** taken along the line **C-C** of FIG. **1b****.**
FIG. **2** is a cross section of the frame of FIGS. **1a, 1b,** and **1c****.**
FIG. **3** is a cross section of one end of the tray of FIGS. **1a, 1b,** and **1c****.**
FIG. **4** is a cross section of the assembled frame and tray of FIGS. **2** and **3** arranged to accommodate a gel beneath the electrode.
FIG. **5** is a cross section of the assembled frame and tray of FIGS. **2** and **3** arranged to accommodate a gel above the electrode.
FIG. **6** is a perspective view of a second example of a tray and frame assembly in accordance with the present invention.
FIG. **7** is a front view of a center section of one of the frames of the assembly of FIG. **6**.
FIG. **8** is a longitudinal cross section of a frame and one end of the tray of the assembly of FIG. **6**.
FIG. **9** is a the same longitudinal cross section as FIG. **8** with the electrode raised.
FIG. **10** is a transverse cross section of a frame and one end of the tray, both of the assembly of FIG. **6**.

### DETAILED DESCRIPTION OF THE INVENTION AND PREFERRED EMBODIMENTS

While the features defining this invention are capable of implementation in a variety of constructions, the invention as a whole will be best understood by a detailed examination of specific embodiments. Two such embodiments are shown in the drawings.

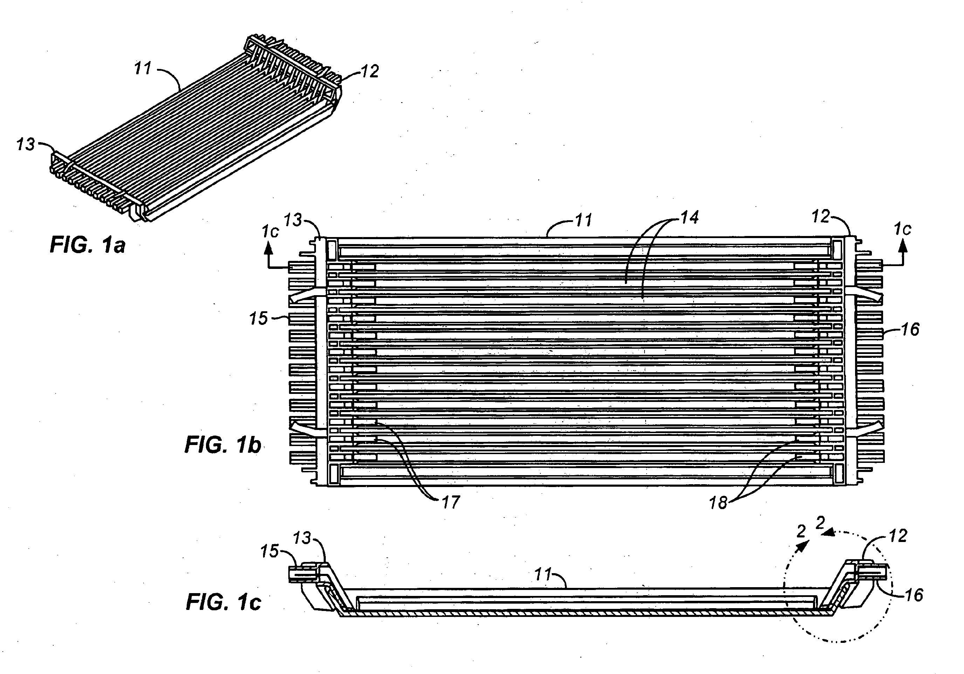

The first embodiment is illustrated in a perspective view in FIG. **1a****,** a top view in FIG. **1b**, and a vertical cross section in FIG. **1c**. The cross section in FIG. **1c** is taken along the line C-C in FIG. **1b**. The tray **11** in these Figures is shown with a frame **12** at one end and a second frame **13** at the opposite end. The tray **11** contains twelve troughs **14**, which are elongate and parallel and sized to accommodate one IPG strip each. Each frame extends the full width of the tray, spanning all troughs. Each frame **12, 13** contains a set of plugs **15, 16** for external electrical connections, one plug for each of the twelve troughs, and a set of electrodes, one for each of the twelve troughs, to contact the gels. The flat inner ends **17, 18** of the electrodes are either metal tabs or plastic tabs with metal wires or strips fixed to both surfaces of the tabs, and serve as the contact electrodes for the gel strips.

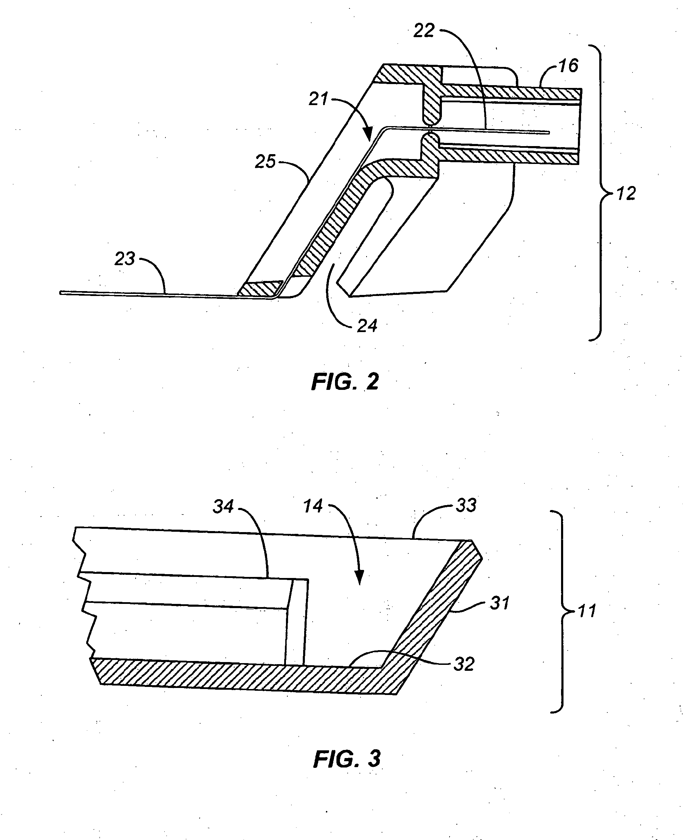

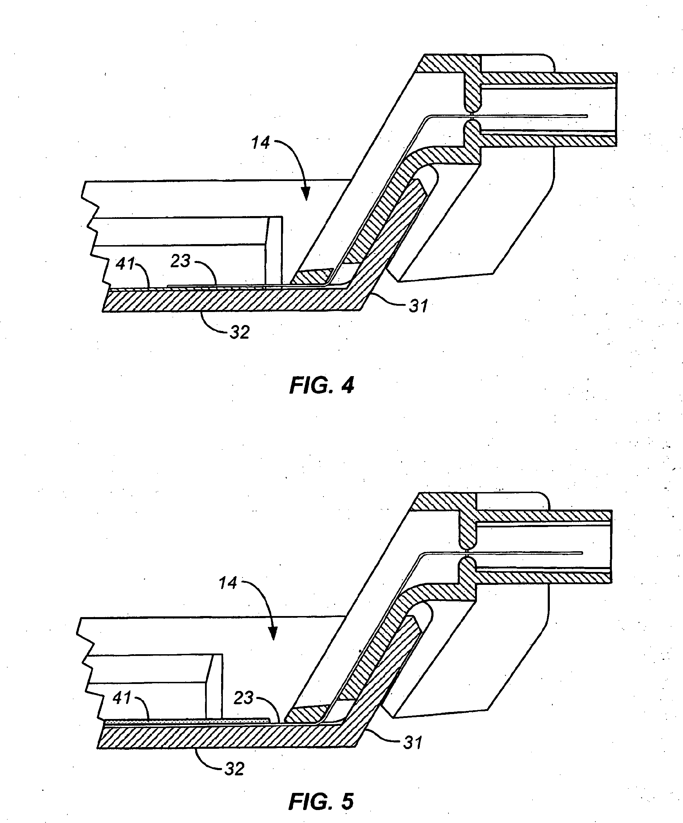

An enlarged view of one of the frames is shown in FIG. **2**, an enlarged view of the end of the tray where the frame is inserted is shown in FIG. **3**, and enlarged views of the frame and tray together are in FIGS. **4** and **5**. These views correspond to the portion of FIG. **1c** indicated by the dashed circle **2** and are all cross sections taken along the same plane as that of FIG. **1c**.

In the cross section of the frame **12** in FIG. **2**, the electrode **21** extends from an outer end **22** inside the plug **16** to its inner end **23**, which in this case is a narrow flat metal tab with exposed upper and lower surfaces. A single transverse slot **24** in the frame (*i.e.*, transverse to the lengthwise direction of the trough and of the electrode **21**) fits over the transverse end wall (shown in FIG. **3** and discussed below) of the tray. Adjacent electrodes are separated by barriers, one of which **25** is visible in FIG. **2**. Each barrier has a longitudinal slot (not visible in FIG. **2**) that fits over the partitions in the tray that divide the tray into troughs.

The tray **11** is shown in cross section in FIG. **3**. The Figure shows the interior of a single trough **14** of the tray, terminating at its end in the transverse end wall **31**, which is received within the transverse slot **24** of the frame. The trough is closed at the bottom by a floor **32**, and adjacent troughs are separated by partitions **33** (referred to in the preceding paragraph) of which one is shown. A reinforcing structure **34** is molded onto the inner surface of the partition **33.**

FIGS. **4** and **5** show the assembled tray and frame with an IPG strip **41**. In FIG. **4**, the IPG strip **41** has been placed on the floor of the trough **14** before the frame has been placed in position. The inner end **23** of the electrode therefore lies above the IPG strip, contacting the upper side of the strip, and the IPG strip **41** has been placed with the gel side up to contact the electrode. In FIG. **5**, the frame has been placed in the tray before the IPG strip **41**, and the IPG strip has been placed over the electrode with the gel side down. The inner end **23** of the electrode thus lies below the IPG strip and the gel side of the IPG strip again contacts the electrode. The frame resides slightly lower in the tray in the FIG. **5** position than in the FIG. **4** position. The tray and frame combination can thus accommodate gels of any thickness and can be used with the electrode contacting either the top of the gel or the bottom of the gel.

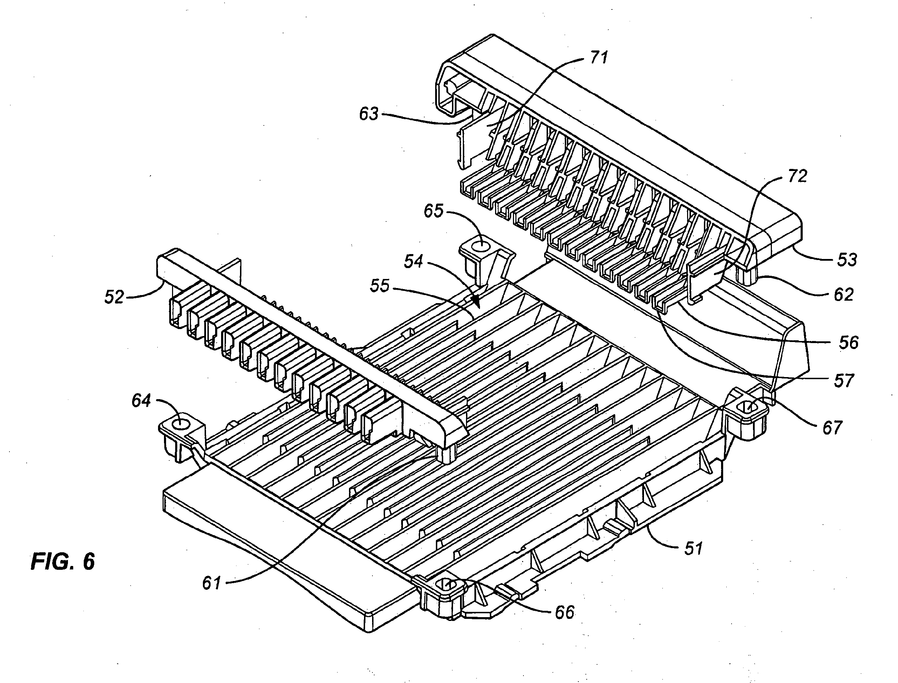

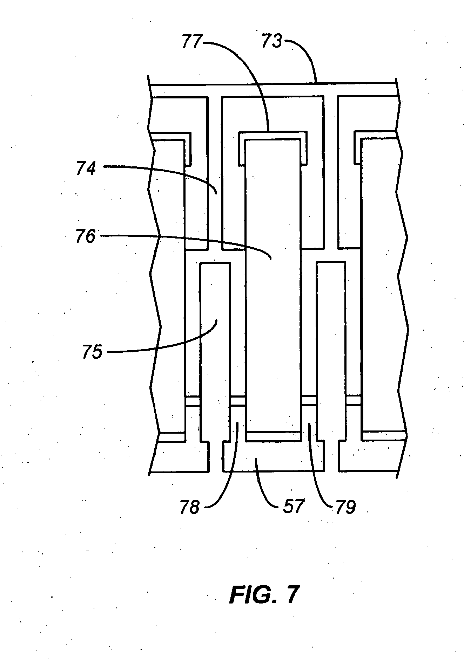

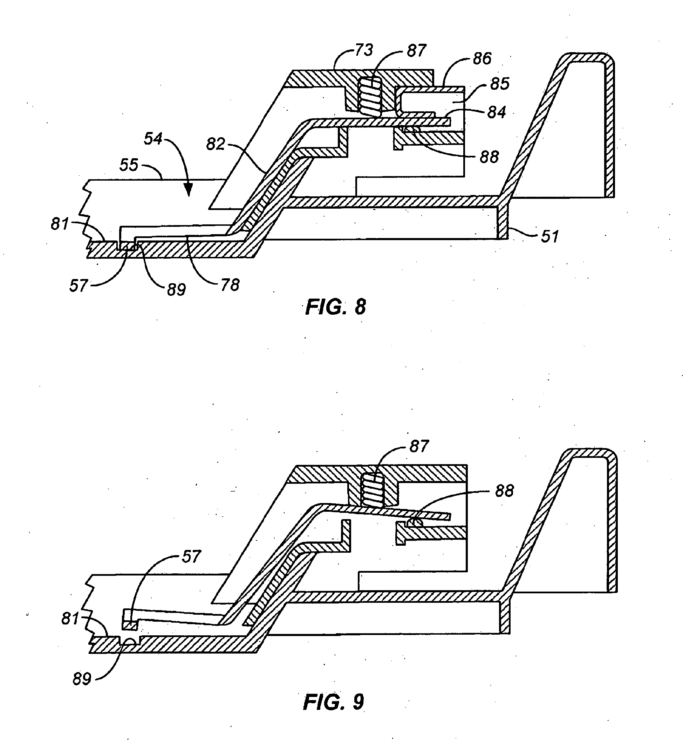

The second embodiment is illustrated in the succeeding Figures. FIG. **6** is a perspective view of the tray **51** and two frames **52, 53** raised above the tray for visibility. Like the tray **11** of the embodiment of FIG. **1**, the tray **51** in the embodiment of FIG. **6** has twelve troughs **54**, separated by partitions **55**, and each frame has twelve electrodes **56** of which only those on the right frame are visible. A feature that distinguishes the frames in this embodiment from those of the embodiment of FIGS. **1-5** is the configurations of the inner ends of the electrodes **56**. In the embodiment of FIG. **6**, each individual electrode is a loop at its inner end, terminating in a right-angle downward bed and a crossbar **57**. The gel strip fits between the two longitudinal side arms of the loop. The crossbar **57** spans the width of the gel and serves as the electrical contact with the gel, either above or below the gel. Further views of the loop and crossbar appear in FIGS. **7** and **8**, discussed below.

Another feature that distinguishes the frames in this embodiment from those of the embodiment of FIGS. **1-5** is the inclusion of positioning posts on the frames and complementary holes in the tray to receive the posts. Only the posts **61, 62** on one end of each of the frames are fully visible in this perspective view, while further posts, one of which **63** is partially visible, are similarly situated on the opposite ends of the frames. All four holes **64, 65, 66, 67** in the tray are visible, one hole in direct alignment with each of the four posts. To ensure proper placement of the frames in the tray, one post on each frame has a square cross section (with rounded corners) while the other has a circular cross section, the corresponding holes having complementary cross sections to allow insertion of only the proper post.

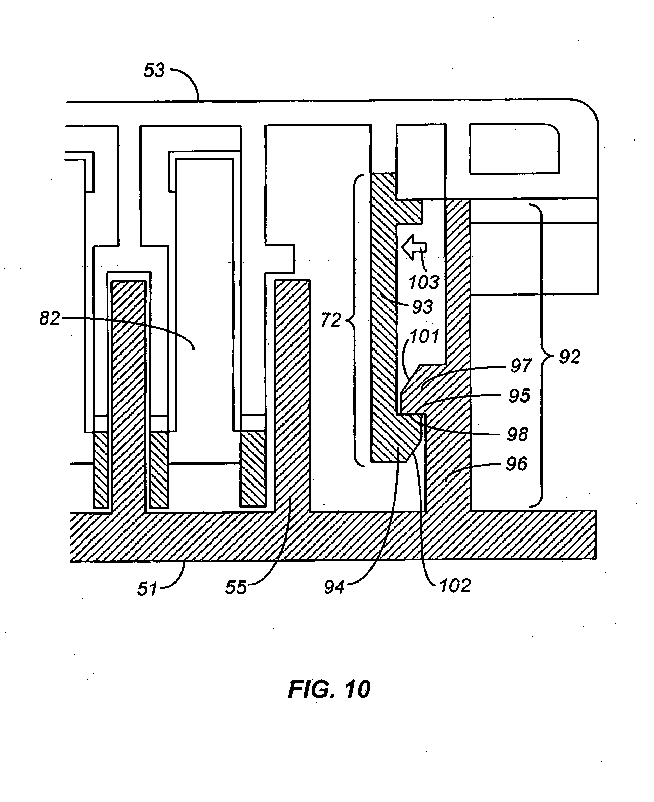

A third feature distinguishing the frames in this embodiment from those of the embodiment of FIGS. **1-5** is a system of latches that secure the frames in a fixed position the tray. Portions **71, 72** of two of the latches are visible in the Figure. An enlarged cross section of an entire single latch is shown in FIG. **10** and discussed below.

FIG. **7** shows a center section of one of the frames **52** in a view facing the inner ends of the electrodes. The body **73** of the frame is of molded plastic or any other non-conducting and chemically inert material, and contains barriers **74** that separate the electrodes. Slots **75** in the barriers fit over the partitions **55** between the troughs in the tray (FIG. **6**). The electrodes **76** are flat metal strips passing through apertures **77** in the frame body. Each electrode ends in an extended loop formed by longitudinal side arms **78, 79** that are joined at their inner ends by the crossbar **57**.

Longitudinal cross sections of one end of the tray with a frame in place are presented in FIGS. **8** and **9****.** Referring to FIG. **8**, the cross section is cut longitudinally through a single trough **54** of the tray **51**, with the floor **81** of the trough and the partition **55** separating the troughs both visible. A single electrode **82** is visible, supported by the body **73** of the frame. The inner end of the electrode is a loop formed of parallel side arms, one of which **78** is visible, terminating in the crossbar **57**. The outer end of the electrode is an exposed tip **84** which extends into the interior of a hollow cavity **85** where the tip is accessible for contact with an external electric lead. As noted above, individual electrode tips can be configured as individual plugs for individual control of the voltage. Alternatively, the electrode tips on one of the frames can be joined to a common conductive strip **86**, as shown in FIG. **8**. The common strip **86** can then be connected to an external lead.

The electrode **82** in this embodiment is resiliently mounted to the frame body in a manner that urges the crossbar **57** at the electrode's inner end toward the floor **81** of the trough. This is accomplished by a coil spring **87** in the frame body and a pivot point **88**. The coil spring **87** urges the inner end of the electrode down, and electrode can be pressed upward against the spring by a gel strip underneath the crossbar **57**. In FIG. **8**, the electrode is in its lowest position, suitable for a gel strip placed above the electrode, and the crossbar **57** rests inside an indentation **89** in the floor **81** of the trough. In FIG. **9**, the electrode has been pivoted around the pivot point **88** to raise the crossbar **57** above the floor **81**, leaving a gap for the gel strip (not shown) which will have been placed in the trough before the frame is lowered into the tray. Since the gel strip itself pushes upward on the crossbar **57**, the crossbar height is determined by the thickness of the gel strip and will adjust accordingly.

The latch system in this embodiment contains four latches, one to secure each end of each frame to one of the four corners of the tray. One of the latches is shown in FIG. **10**. the latch consist of two parts, an upper part **72** (also visible in FIG. **6**) near the end of the frame **53** and a lower part **92** cross section of the portion of the tray **51** that contains the of the latch. The upper part **72** includes a downwardly extending arm **93** whose lower end has a lateral projection **94** that forms a shoulder **95** while the lower part **92** is an upwardly extending wall **96** that runs parallel to the partitions **55** that separate the troughs. The upwardly extending wall **96** has a lateral protrusion **97** that forms an inverted shoulder **98** that engages the shoulder **94** on the upper portion. The two protrusions have sloping surfaces **101, 102** opposite the shoulders to allow the parts to slide together to make a snap fit. Release of the latch is achieved by finger pressure on the downwardly extending arm **93** of the upper part in the direction of the arrow **103** to disengage the shoulders.

Using either of these two constructions, *i.e*., that of FIGS. **1-5** and that of FIGS. **6-10****,** isoelectric focusing can be performed on two or more samples either simultaneously or independently, and the electric potential applied across any single IPG strip can be selected independently of all other IPG strips by using differential potentials for each different pair of electrodes. The samples can be applied to the strips either before or after the strips have been placed inside the troughs.

In the claims appended hereto, the term "a" or "an" is intended to mean "one or more." The term "comprise" and variations thereof such as "comprises" and "comprising," when preceding the recitation of a step or an element, are intended to mean that the addition of further steps or elements is not excluded from the scope of the claim.

## Claims

1. Apparatus for isoelectric focusing in a plurality of gel strips, said apparatus comprising:
a frame (12) with a plurality of electrodes (21) mounted thereto, each electrode (21) terminating in an inner electrode end (23) having upper and lower surfaces both of which comprise exposed electrode material; and
a tray (11) having a plurality of troughs (14), each said trough (14) having a floor (32), said troughs (14) sized and spaced to receive one gel strip (41) per trough (14) and one inner electrode end (23) lying either above or below said gel strip (41);
wherein when said inner electrode ends (23) are received within said troughs (14), **characterized in that** said inner electrode end (23) of each of said electrodes (21) is variable in height between a zero height in which said inner electrode end (23) directly contacts said floor (32) while allowing a gel strip (41) to rest on said upper surface and a finite height in which said lower surface is raised above said floor by a distance sufficient to receive a gel strip (41) with said lower surface contacting an upper side of said gel strip (41); and **in that**
said electrodes (21) are mounted to said frame (12) in a manner allowing for control of the height of each inner electrode end (23) independently of all other inner electrode ends (23).

2. The apparatus of claim 1, wherein said electrodes (21) are mounted to said frame (12) in a manner for resiliently urging said inner electrode ends (23) toward said floors (32).

3. The apparatus of claim 1, wherein each said electrode (21) is pivotally mounted to said frame (12) and said frame (12) comprises spring means resiliently urging each said inner electrode end (23) toward a floor of said tray (11).

4. The apparatus of claim 1, wherein each said trough (14) terminates in an end wall, and said apparatus further comprises a slot (24) in said frame sized to receive said end wall, said slot (24) and said end wall being configured to maintain said inner electrode ends (23) parallel to said floors (32) of said troughs (14).

5. The apparatus of claim 1, further comprising a latch connection (72) for joining said frame (12) to said tray (11) that is releasable by finger pressure.

6. The apparatus of claim 1, comprising a pair of said frames (12), and further comprising means for joining one of said frames (12) to each of two ends of said tray (11) at opposing ends of said troughs (14).

7. A method for performing isoelectric focusing on a plurality of samples in individual isoelectric focusing gel strips (41) either simultaneously or independently in a single gel strip holder, said method comprising:
(a) placing said gel strips (41) in troughs (14) of a tray (11) having a plurality of troughs (14), each trough (14) having a floor (32) and sized to receive one gel strip (41) resting on said floor (32);
(b) either before or after step (a), applying said samples to said gel strips (41), with one sample per gel strip (41);
(c) either before, after, or between steps (a) and (b), placing a frame (12) on said tray, said frame having a plurality of electrodes (21) mounted thereto, each electrode (21) terminating in an inner electrode end (23) having parallel upper and lower surfaces both of which comprise exposed electrode material, said inner electrode ends (23) sized and spaced to fit within said troughs (14) with one inner electrode end (23) per trough (14) and both upper and lower surfaces within said trough (14), such that upon completion of steps (a), (b), and (c) each said gel strip (41) is in direct contact with a single inner electrode end (23), wherein said electrodes are mounted to said frame (12) in a manner allowing the height of each inner electrode end (23) above the floor (32) of the trough (14) in which said inner electrode end (23) is inserted to vary individually; and
(d) imposing an electric potential across each said strip through said contact between said strip and said inner electrode end (23) to separate solutes in said samples along said gel strips (41) by isoelectric focusing.

8. The method of claim 7, wherein step (c) is performed before step (a), and step (a) comprises placing said gel strips (41) above said inner electrode ends (23).

9. The method of claim 7, wherein step (a) is performed after step (c), and step (c) comprises placing said inner electrode ends (23) above said gel strips (41).

10. The method of claim 7, wherein said electrodes (21) are mounted to said frame (12) by a mounting that resiliently urges said inner electrode ends (23) toward said floors (32).

11. The method of claim 7, wherein said electrodes (21) are pivotally mounted to is said frame (12) and said frame comprises spring means resiliently urging each said inner electrode end (23) toward a floor (32) of said tray (11).

12. The method of claim 7, wherein step (c) comprises placing two said frames (12) in said tray (11), one frame (12) at each of two ends of said tray (11) at opposing ends of said troughs (14), thereby placing each end of each said gel strip (41) in direct contact with an inner electrode end (23) on one of said frames (12), and step (d) comprises imposing electric potentials between inner electrode ends (23) at opposing ends of each gel strip (41).

## Patentansprüche

1. Vorrichtung zur isoelektrischen Fokussierung in einer Vielzahl von Gelstreifen, wobei die Vorrichtung umfasst:
einen Rahmen (12) mit einer Vielzahl von darauf montierten Elektroden (21), wobei jede Elektrode (21) in einem inneren Elektrodenende (23) endet, das eine obere und eine untere Oberfläche aufweist, die beide exponiertes Elektrodenmaterial umfassen; und
eine Wanne (11) mit einer Vielzahl von Trögen (14), wobei jeder Trog (14) einen Boden (32) aufweist, wobei die Tröge (14) so groß und so weit voneinander entfernt sind, dass sie einen Gelstreifen (41) pro Trog (14) aufnehmen können, und ein inneres Elektrodenende (23) entweder oberhalb oder unterhalb des Gelstreifens (41) liegt;
**dadurch gekennzeichnet, dass** das innere Elektrodenende (23) jeder der Elektroden (21), wenn die inneren Elektrodenenden (23) innerhalb der Tröge (14) aufgenommen sind, in der Höhe zwischen einer Nullhöhe, bei der das innere Elektrodenende (23) direkt in Kontakt mit dem Boden (32) steht, während ein Gelstreifen (41) auf der oberen Oberfläche aufliegen kann, und einer endlichen Höhe, bei der die untere Oberfläche ausreichend weit über den Boden erhöht ist, dass ein Gelstreifen (41) aufgenommen werden kann, wobei die untere Oberfläche mit einer oberen Seite des Gelstreifens (41) in Kontakt steht, variabel ist; und
dadurch, dass die Elektroden (21) in einer Weise auf dem Rahmen (12) montiert sind, die es ermöglicht, die Höhe jedes inneren Elektrodenendes (23) unabhängig von allen anderen inneren Elektrodenenden (23) zu steuern.

2. Vorrichtung gemäß Anspruch 1, wobei die Elektroden (21) in einer Weise auf dem Rahmen (12) montiert sind, die die inneren Elektrodenenden (23) elastisch zu den Böden (32) hin drückt.

3. Vorrichtung gemäß Anspruch 1, wobei jede Elektrode (21) schwenkbar auf dem Rahmen (12) montiert ist und der Rahmen (12) Federeinrichtungen umfasst, die jedes innere Elektrodenende (23) elastisch zu einem Boden der Wanne (11) hin drücken.

4. Vorrichtung gemäß Anspruch 1, wobei jeder Trog (14) in einer Endwand endet und die Vorrichtung weiterhin einen Schlitz (24) in dem Rahmen umfasst, der so groß ist, dass er die Endwand aufnehmen kann, wobei der Schlitz (24) und die Endwand so konfiguriert sind, dass die inneren Elektrodenenden (23) parallel zu den Böden (32) der Tröge (14) gehalten werden.

5. Vorrichtung gemäß Anspruch 1, die weiterhin eine Riegelverbindung (72) zum Verbinden des Rahmens (12) mit der Wanne (11), die durch Fingerdruck lösbar ist, umfasst.

6. Vorrichtung gemäß Anspruch 1, die ein Paar der Rahmen (12) umfasst und weiterhin Einrichtungen umfasst, um einen der Rahmen (12) mit jedem von zwei Enden der Wanne (11) an entgegengesetzten Enden der Tröge (14) zu verbinden.

7. Verfahren zur Durchführung einer isoelektrischen Fokussierung mit einer Vielzahl von Proben in individuellen Gelstreifen (41) für die isoelektrische Fokussierung entweder gleichzeitig oder unabhängig voneinander in einem einzigen Gelstreifenhalter, wobei das Verfahren umfasst:
(a) Platzieren von Gelstreifen (41) in Trögen (14) einer Wanne (11), die eine Vielzahl von Trögen (14) aufweist, wobei jeder Trog (14) einen Boden (32) aufweist und so groß ist, dass er einen Geistreifen (41), der auf dem Boden (32) aufliegt, aufnehmen kann;
(b) entweder vor oder nach Schritt (a) Auftragen der Proben auf die Gelstreifen (41) mit einer Probe pro Gelstreifen (41);
(c) entweder vor, nach oder zwischen den Schritten (a) und (b) Platzieren eines Rahmens (12) auf der Wanne, wobei der Rahmen eine Vielzahl von darauf montierten Elektroden (21) aufweist, wobei jede Elektrode (21) in einem inneren Elektrodenende (23) endet, das parallel zueinander eine obere und eine untere Oberfläche aufweist, die beide exponiertes Elektrodenmaterial umfassen, wobei die inneren Elektrodenenden (23) so groß und so weit voneinander entfernt sind, dass sie mit einem inneren Elektrodenende (23) pro Trog (14) und sowohl der oberen als auch der unteren Oberfläche innerhalb des Trogs (14) in die Tröge (14) passen, so dass sich nach Beendigung der Schritte (a), (b) und (c) jeder Gelstreifen (41) in direktem Kontakt mit einem einzigen inneren Elektrodenende (23) befindet, wobei die Elektroden so auf dem Rahmen (12) montiert sind, dass die Höhe jedes inneren Elektrodenendes (23) über dem Boden (32) des Trogs (14), in den das innere Elektrodenende (23) eingesetzt wird, einzeln variiert werden kann; und
(d) Anlegen einer elektrischen Spannung an jeden Streifen über den Kontakt zwischen dem Streifen und dem inneren Elektrodenende (23), um gelöste Stoffe in den Proben entlang der Gelstreifen (41) durch isoelektrische Fokussierung zu trennen.

8. Verfahren gemäß Anspruch 7, wobei Schritt (c) vor Schritt (a) durchgeführt wird und Schritt (a) das Platzieren der Gelstreifen (41) über den inneren Elektrodenenden (23) umfasst.

9. Verfahren gemäß Anspruch 7, wobei Schritt (a) nach Schritt (c) durchgeführt wird und Schritt (c) das Platzieren der inneren Elektrodenenden (23) über den Gelstreifen (41) umfasst.

10. Verfahren gemäß Anspruch 7, wobei die Elektroden (21) durch eine Montierung, die die inneren Elektrodenenden (23) elastisch zu den Böden (32) hin drückt, auf dem Rahmen (12) montiert sind.

11. Verfahren gemäß Anspruch 7, wobei die Elektroden (21) schwenkbar auf dem Rahmen (12) montiert sind und der Rahmen Federeinrichtungen umfasst, die das innere Elektrodenende (23) elastisch zu einem Boden (32) der Wanne (11) hin drücken.

12. Verfahren gemäß Anspruch 7, wobei Schritt (c) das Platzieren von zweien der Rahmen (12) in der Wanne (11), einen Rahmen (12) an jedem von zwei Enden der Wanne (11) an entgegengesetzten Enden der Tröge (14), umfasst, wodurch jedes Ende jedes Gelstreifens (41) in direktem Kontakt mit einem inneren Elektrodenende (23) an einem der Rahmen (12) platziert wird, und Schritt (d) das Anlegen elektrischer Spannungen zwischen inneren Elektrodenenden (23) an entgegengesetzten Enden jedes Gelstreifens (41) umfasst.

## Revendications

1. Appareil pour focalisation isoélectrique dans une pluralité de bandes de gel, ledit appareil comprenant :
un cadre (12) sur lequel sont montées une pluralité d'électrodes (21), chaque électrode (21) se terminant en une extrémité d'électrode interne (23) ayant des surfaces supérieure et inférieure comprenant toutes les deux un matériau d'électrode exposé ; et
un plateau (11) ayant une pluralité de cannelures (14), chacune desdites cannelures (14) ayant un plancher (32), lesdites cannelures (14) étant dimensionnées et espacées de façon à recevoir une bande de gel (41) par cannelure (14) et une extrémité d'électrode interne (23) se trouvant au-dessus ou en dessous de ladite bande de gel (41) ;
dans lequel lesdites extrémités d'électrodes internes (23) sont logées à l'intérieur desdites cannelures (14),
**caractérisé en ce que** ladite extrémité d'électrode interne (23) de chacune desdites électrodes (21) est variable en hauteur entre une hauteur nulle à laquelle ladite extrémité d'électrode interne (23) entre directement en contact avec ledit plancher (32) tout en permettant à une bande de gel (41) de reposer sur ladite surface supérieure et une hauteur finie à laquelle ladite surface inférieure est élevée au-dessus dudit plancher d'une distance suffisante pour recevoir une bande de gel (41) avec ladite surface inférieure entrant en contact avec une face supérieure de ladite bande de gel (41) ; et **en ce que**
lesdites électrodes (21) sont montées sur ledit cadre (12) d'une manière qui permet une régulation de la hauteur de chaque extrémité d'électrode interne (23) indépendamment de toutes les autres extrémités d'électrodes internes (23).

2. Appareil selon la revendication 1, dans lequel lesdites électrodes (21) sont montées sur ledit cadre (12) d'une manière permettant de pousser de façon résiliente lesdites extrémités d'électrodes internes (23) vers lesdits planchers (32).

3. Appareil selon la revendication 1, dans lequel chacune desdites électrodes (21) est montée de manière pivotante sur ledit cadre (12) et ledit cadre (12) comprend des moyens de ressort poussant de façon résiliente chaque extrémité d'électrode interne (23) vers un plancher dudit plateau (11).

4. Appareil selon la revendication 1, dans lequel chaque cannelure (14) se termine en une paroi d'extrémité, et ledit appareil comprend en outre une fente (24) dans ledit cadre, dimensionnée de manière à recevoir ladite paroi d'extrémité, ladite fente (24) et ladite paroi d'extrémité étant configurées pour maintenir lesdites extrémités d'électrodes internes (23) parallèles auxdits planchers (32) desdites cannelures (14).

5. Appareil selon la revendication 1, comprenant en outre une connexion de verrouillage (72) pour réunir ledit cadre (12) audit plateau (11), qui est libérable par une pression des doigts.

6. Appareil selon la revendication 1, comprenant une paire desdits cadres (12), et comprenant en outre des moyens pour réunir l'un desdits cadres (12) à chacune de deux extrémités dudit plateau (11), à des extrémités opposées desdites cannelures (14).

7. Procédé pour exécuter une focalisation isoélectrique sur une pluralité d'échantillons dans des bandes de gel de focalisation isoélectrique individuelles (41), simultanément ou bien indépendamment, dans un seul support de bandes de gel, ledit procédé comprenant les étapes suivantes :
(a) placer lesdites bandes de gel (41) dans des cannelures (14) d'un plateau (11) ayant une pluralité de cannelures (14), chaque cannelure (14) ayant un plancher (32) et étant dimensionnée de manière à recevoir une bande de gel (41) reposant sur ledit plancher (32) ;
(b) avant ou bien après l'étape (a), appliquer lesdits échantillons sur lesdites bandes de gel (41), avec un échantillon par bande de gel (41) ;
(c) avant, après, ou bien entre les étapes (a) et (b), placer un cadre (12) sur ledit plateau, une pluralité d'électrodes (21) étant montée sur ledit plateau, chaque électrode (21) se terminant en une extrémité d'électrode interne (23) ayant des surfaces supérieure et inférieure parallèles comprenant toutes les deux un matériau d'électrode exposé, lesdites extrémités d'électrodes internes (23) étant dimensionnées et espacées de manière à s'ajuster dans lesdites cannelures (14) avec une extrémité d'électrode interne (23) par cannelure (14) et les surfaces supérieure et inférieure toutes les deux à l'intérieur de ladite cannelure (14), de telle sorte que, lors de l'achèvement des étapes (a), (b) et (c), chacune desdites bandes de gel (41) soit en contact direct avec une seule extrémité d'électrode interne (23), où lesdites électrodes sont montées sur ledit cadre (12) d'une manière qui permet de faire varier individuellement la hauteur de chaque extrémité d'électrode interne (23) au-dessus du plancher (32) de la cannelure (14) dans laquelle est insérée ladite extrémité d'électrode interne (23) ; et
(d) imposer un potentiel électrique aux bornes de chacune desdites bandes à travers ledit contact entre ladite bande et ladite extrémité d'électrode interne (23) pour séparer par focalisation isoélectrique les solutés présents dans lesdits échantillons le long desdites bandes de gel (41).

8. Procédé selon la revendication 7, dans lequel l'étape (c) est exécutée avant l'étape (a), et l'étape (a) comprend la mise en place desdites bandes de gel (41) au-dessus desdites extrémités d'électrodes internes (23).

9. Procédé selon la revendication 7, dans lequel l'étape (a) est exécutée après l'étape (c), et l'étape (c) comprend la mise en place desdites extrémités d'électrodes internes (23) au-dessus desdites bandes de gel (41).

10. Procédé selon la revendication 7, dans lequel lesdites électrodes (21) sont montées sur ledit cadre (12) par un montage qui pousse de façon résiliente lesdites extrémités d'électrodes internes (23) vers lesdits planchers (32).

11. Procédé selon la revendication 7, dans lequel lesdites électrodes (21) sont montées de manière pivotante sur ledit cadre (12) et ledit cadre comprend des moyens de ressort poussant de façon résiliente chaque extrémité d'électrode interne (23) vers un plancher dudit plateau (11).

12. Procédé selon la revendication 7, dans lequel l'étape (c) comprend la mise en place de deux desdits cadres (12) dans ledit plateau (11), un cadre (12) à chacune de deux extrémités dudit plateau (11), à des extrémités opposées desdites cannelures (14), pour ainsi placer chaque extrémité de chaque bande de gel (41) en contact direct avec une extrémité d'électrode interne (23) sur l'un desdits cadres (12), et l'étape (d) comprend l'application de potentiels électriques entre les extrémités d'électrodes internes (23) aux extrémités opposées de chaque bande de gel (41).
